# EUROPÄISCHE PATENTANMELDUNG

(11) **EP 2 100 883 A1**
(43) Veröffentlichungstag der Anmeldung: **16.09.2009**
(21) Anmeldenummer: 08152532.1
(22) Anmeldetag: 10.03.2008
(51) Int. Cl.: C07D 231/14

(54) **Verfahren zur regioselektiven Synthese von 1-Alkyl-3-haloalkyl-pyrazol-4carbonsäure-Derivaten**

(71) Anmelder: Bayer CropScience AG, 40789 Monheim (DE)
(72) Erfinder: Die Erfindernennung liegt noch nicht vor

(57) **Zusammenfassung**

Die vorliegende Erfindung betrifft ein Verfahren zur regioselektiven Synthese von 1-Alkyl-3-haloalkyl-pyrazol-4-carbonsäure-Derivaten durch Cyclisierung von 2,3-disubstituierten Acrylsäure-Derivaten mit Hydrazinen, in Gegenwart von Carbonylverbindungen.

## Beschreibung

Die vorliegende Erfindung betrifft ein Verfahren zur regioselektiven Synthese von 1-Alkyl-3-haloalkyl-pyrazol-4-carbonsäure-Derivaten durch Cyclisierung von 2,3-disubstituierten Acrylsäure-Derivaten mit Hydrazinen, in Gegenwart von Carbonylverbindungen.

2-Dihalogenacyl-3-dialkylamino-acrylsäureester der Formel II (Y=COOAlk, Z=O) sind wertvolle Zwischenprodukte für die Herstellung von dihalogenmethyl-substituierten Pyrazolylcarbonsäure-Derivaten, die als Vorstufen von fungiziden Wirkstoffen verwendet werden können (vgl. WO 03/070705).

Pyrazolcarbonsäure-Derivate werden üblicherweise hergestellt, indem man Acrylsäure-Derivate, die zwei Abgangsgruppen (Z und A) aufweisen, mit Hydrazinen umsetzt.

Bei der Umsetzung mit den Monoalkylhydrazinen erhält man vorwiegend 1-Alkylpyrazole. Allerdings erfolgt die Cyclisierung oftmals nicht regioselektiv. Folglich werden, in Abhängigkeit von Substrat und Reaktionsbedingungen, die unerwünschten 5-Alkylpyrazole in Mengen zwischen 10 und 80 % gebildet (siehe Schema 1).

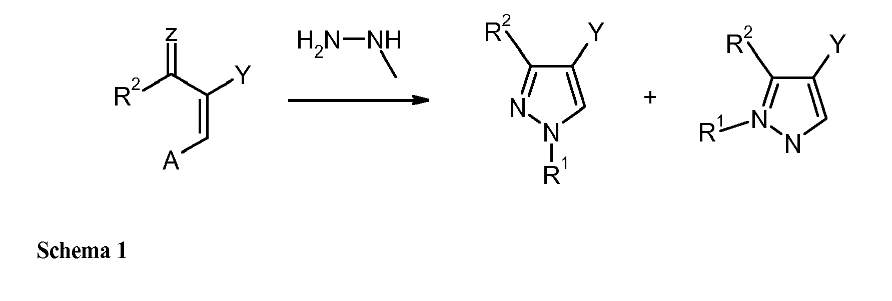

Auch die Synthese von 1-Alkylpyrazolcarbonsäure-Derivaten durch Alkylierung von in 1-Position unsubstituerten Pyrazolderivaten verläuft oftmals unter Bildung beider Regioisomere (siehe Schema 2).

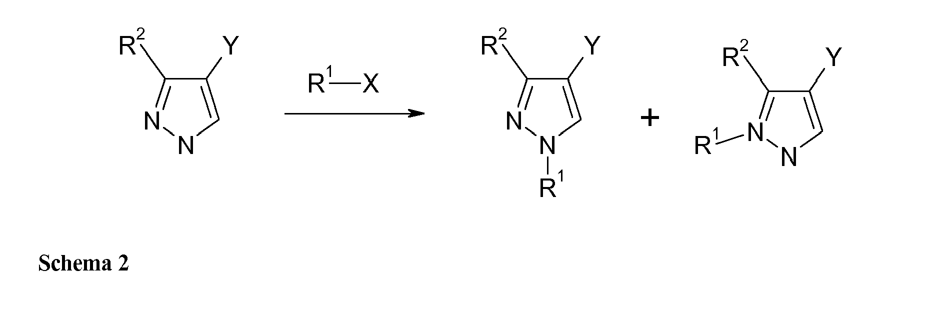

Ein alternativer Weg zur Herstellung von Fluorhaloalkylpyrazolencarbonsäuren ist die Cyclisierung von z. B 4,4-Dichloro-2-[(dimethylamino)methylidene]-3-oxobutanoat mit Alkylhydrazinen, gefolgt von einem Halogenaustausch.

WO 2005/042468 offenbart ein Verfahren zum Herstellen von 2-Dihalogenacyl-3-aminoacrylsäureestern durch Umsetzung von Säurehalogeniden mit Dialkylaminoacrylsäureestern und deren anschließende Cyclisierung mit Alkylhydrazinen

Die bislang unveröffentlichte Europäische Patentanmeldung Nr. 07117232.4 beschreibt ein Verfahren zur Herstellung von HCI-freien 2-Dihalogenacyl-3-amino-acrylsäureestern durch Umsetzung von Säurefluoriden mit Dialkylaminoacrylsäure-Derivaten. Das Verfahren kann in Abwesenheit einer Base durchgeführt werden, wodurch die Abtrennung von Halogenid-Salzen entfällt.

PCT/EP/2007/007377 beschreibt ein Verfahren zum Herstellen von 3-Dihalomethyl-pyrazol-4-carbonsäure-Derivaten durch Umsetzung von α,α-Fluoraminen in Gegenwart von Lewissäuren mit Acrylsäure-Derivaten und deren anschließender Umsetzung mit Alkylhydrazinen.

WO 2006/090778 offenbart ein Verfahren zur Herstellung von 1-Methyl-3-difluormethylpyrazolcarbonsäureestern durch Cyclisierung von 2-Alkoxymethylenfluoracylacetat mit Methylhydrazin in Anwesenheit von Wasser und einer Base. Die Umsetzung wurde in Gegenwart von NaOH oder KOH durchgeführt obwohl unter diesen stark alkalischen Bedingungen teilweise Verseifung von COOEt-Gruppe des Pyrazolrings auch stattfindet.

Die zuvor beschriebenen Verfahren weisen jedoch alle den Nachteil auf, dass die Cyclisierung, selbst bei niedrigen Temperaturen, nur mit unbefriedigender Regioselektivität erfolgt.

Im Lichte des zuvor beschrieben Standes der Technik, liegt der vorliegenden Erfindung die Aufgabe zugrunde, ein Verfahren zur Verfügung zu stellen, dass die zuvor genannten Nachteile nicht aufweist und somit einen regioselektiven Zugang zu 1-Alkyl-3-haloalkyl-pyrazol-4-carbonsäure-Derivaten in hohen Ausbeuten gewährt.

Die zuvor beschriebene Aufgabe wurde gelöst durch ein Verfahren zum Herstellen von 1-Alkyl-3-haloalkyl-pyrazol-4-carbonsäure-Derivaten der Formel (I) 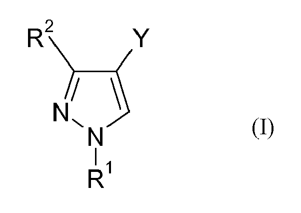 in welcher
- R¹: ausgewählt ist aus C₁-C₄-Alkylgruppen,
- R²: ausgewählt ist aus C₁-C₄-Alkylgruppen, die mit einem, zwei oder drei Halogenatomen, ausgewählt aus F, Cl und Br oder einer CF₃-Gruppe substituiert sein können;
- Y: ausgewählt ist aus der Gruppe bestehend aus (C=O)OR³, CN und (C=O)NR⁴R⁵, wobei R³, R⁴ und R⁵ unabhängig voneinander ausgewählt sind aus C₁₋₁₂-Alkyl-, C₃₋₋₈- Cycloalkyl-, C₆₋₁₈-Aryl-, C₇₋₁₉-Arylalkyl- oder C₇₋₁₉-Alkylarylresten oder wobei R⁴ und R⁵ gemeinsam mit dem N-Atom an das sie geknüpft sind einen fünf oder sechsgliedrigen Ring bilden können;
umfassend die Umsetzung eines 2-acylierten Acrylsäure-Derivats der Formel (II), 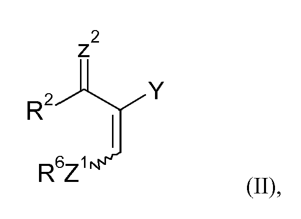 in welcher
- Z¹ und Z²: unabhängig voneinander ausgewählt sind aus O und S,
- R⁶: ausgewählt ist aus C₁₋₁₂-Alkylgruppen;
mit einem N-Alkyl-Hydrazin der Formel (III) 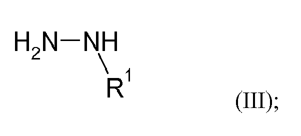 in Gegenwart einer Carbonylverbindung der Formel IV 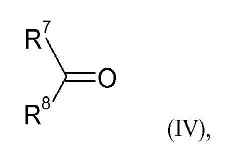 in welcher
- R⁷, R⁸: unabhängig voneinander ausgewählt sind aus H, C₁₋₁₂-Alkyl-, C₃₋₈-Cycloalkyl-, C₆₋₁₈-Aryl-, C₇₋₁₉-Arylalkyl- oder C₇₋₁₉-Alkylarylresten.

Überraschenderweise lassen sich die 1-Alkyl-3-haloalkyl-pyrazol-4-carbonsäure-Derivate der Formel (I) unter den erfindungsgemäßen Bedingungen mit guten Ausbeuten, Regioselektivitäten und in hoher Reinheit herstellen, womit das erfindungsgemäße Verfahren die oben genannten Nachteile der im Stand der Technik vorbeschriebenen Herstellungsverfahren überwindet.

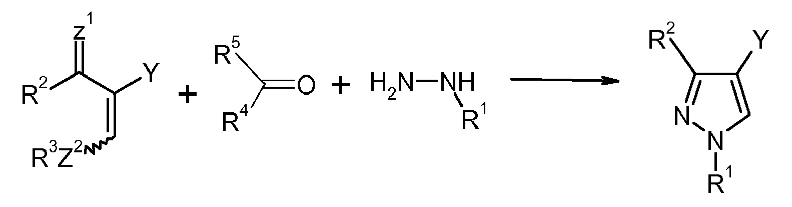

### Allgemeine Definitionen

Im Zusammenhang mit der vorliegenden Erfindung umfasst der Begriff Halogene soweit nicht anders definiert, solche Elemente, die ausgewählt sind aus der Gruppe bestehend aus Fluor, Chlor, Brom und Iod, wobei Fluor, Chlor und Brom bevorzugt und Fluor und Chlor besonders bevorzugt verwendet werden.

Gegebenenfalls substituierte Gruppen können einfach oder mehrfach substituiert sein, wobei bei Mehrfachsubstitutionen die Substituenten gleich oder verschieden sein können.

Mit einem oder mehreren Halogenatomen substituierte Alkyl-Gruppen sind beispielsweise ausgewählt aus Trifluormethyl (CF₃), Difluormethyl (CHF₂), CF₃CH₂, ClCH₂, CF₃CCl₂, CF₃CFH.

Alkyl-Gruppen sind im Zusammenhang mit der vorliegenden Erfindung, soweit nicht abweichend definiert, lineare, verzweigte oder ringförmige gesättigte Kohlenwasserstoff-Gruppen. Die Definition C₁-C₁₂-Alkyl umfasst den größten hierin definierten Bereich für einen Alkyl-Gruppe. Im Einzelnen umfasst diese Definition beispielsweise die Bedeutungen Methyl, Ethyl, n-, iso-Propyl, n-, iso-, sec- und t-Butyl, n-Pentyl, n-Hexyl, 1,3-Dimethylbutyl, 3,3-Dimethylbutyl, n-Heptyl, n-Nonyl, n-Decyl, n-Undecyl, n-Dodecyl. Die Definition C₃-C₁₂-Cycloalkyl umfasst beispielsweise Cyclopropyl, Cyclobutyl, Cyclopentyl, Cyclohexyl, Cycloheptyl und Cyclooctyl.

Aryl-Gruppen sind im Zusammenhang mit der vorliegenden Erfindung, soweit nicht abweichend definiert, aromatische Kohlenwasserstoff-Gruppen, die ein, zwei oder mehrere Heteroatome, die ausgewählt sind aus O, N, P und S, aufweisen können. Die Definition C₅₋₁₈-Aryl umfasst den größten hierin definierten Bereich für eine Aryl-Gruppe mit 5 bis 18 Gerüst-Atomen, wobei die C-Atome gegen Heteroatome ausgetauscht sein können. Im Einzelnen umfasst diese Definition beispielsweise die Bedeutungen Cyclopentadienyl, Phenyl, Cycloheptatrienyl, Cyclooctatetraenyl, Naphthyl und Anthracenyl; 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5- Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl und 1,3,4-Triazol-2-yl; 1-Pyrrolyl, 1-Pyrazolyl, 1,2,4-Triazol-1-yl, 1-Imidazolyl, 1,2,3-Triazol-1-yl, 1,3,4-Triazol-1-yl; 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl und 1,2,4-Triazin-3-yl.

Arylalkyl-Gruppen (Aralkyl-Gruppen) sind im Zusammenhang mit der vorliegenden Erfindung, soweit nicht abweichend definiert, durch Aryl-Gruppen substituierte Alkyl-Gruppen, die eine C₁₋₈-Alkylenkette aufweisen können und im Arylgerüst ein oder mehrere Heteroatome, die ausgewählt sind aus O, N, P und S, aufweisen können. Die Definition C₇₋₁₉-Aralkyl-Gruppe umfasst den größten hierin definierten Bereich für einen Arylalkyl-Gruppe mit insgesamt 7 bis 19 Atomen in Gerüst und Alkylenkette. Im Einzelnen umfasst diese Definition beispielsweise die Bedeutungen Benzyl- und Phenylethyl-.

Alkylaryl-Gruppen (Alkaryl-Gruppen) sind im Zusammenhang mit der vorliegenden Erfindung, soweit nicht abweichend definiert, durch Alkyl-Gruppen substituierte Aryl-Gruppen, die eine C₁₋₈-Alkylenkette aufweisen können und im Arylgerüst ein oder mehrere Heteroatome, die ausgewählt sind aus O, N, P und S, aufweisen können. Die Definition C₇₋₁₉-Alkylaryl-Gruppe umfasst den größten hierin definierten Bereich für einen Alkylaryl-Gruppe mit insgesamt 7 bis 19 Atomen in Gerüst und Alkylenkette. Im Einzelnen umfasst diese Definition beispielsweise die Bedeutungen Tolyl-, 2,3-, 2,4-, 2,5-, 2,6-, 3,4- oder 3,5-Dimethylphenyl.

Die erfindungsgemäßen Verbindungen können gegebenenfalls als Mischungen verschiedener möglicher isomerer Formen, insbesondere von Stereoisomeren, wie z.B. E- und Z-, threo- und erythro-, sowie optischen Isomeren, gegebenenfalls aber auch von Tautomeren vorliegen. Es werden sowohl die E- als auch die Z-Isomeren, wie auch die threo- und erythro-, sowie die optischen Isomeren, beliebige Mischungen dieser Isomeren, sowie die möglichen tautomeren Formen offenbart und beansprucht.

Die gemäß dem erfindungsgemäßen Verfahren erhältlichen 1-Alkyl-3-haloalkyl-pyrazol-4-carbonsäure-Derivaten sind Verbindungen der Formel (I)

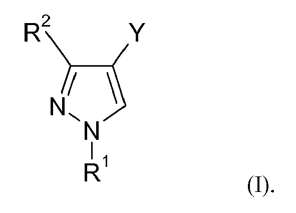

Die Reste in Formel (I) haben erfindungsgemäß die folgenden Bedeutungen:
- R¹: ist ausgewählt aus C₁-C₄-Aklgruppen,
- R²: ist ausgewählt aus C₁-C₄-Alkylgruppen, die mit einem, zwei oder drei Halogenatomen, ausgewählt aus F, Cl und Br oder einer CF₃-Gruppe substituiert sein können;
- Y: ist ausgewählt aus der Gruppe bestehend aus (C=O)OR³, CN und (C=O)NR⁴R^{5,} wobei R³, R⁴ und R⁵ unabhängig voneinander ausgewählt sind aus C₁₋₁₂-Alkyl-, C₃₋₈-Cycloalkyl- , C₆₋₁₈-Aryl-, C₁₋₁₉-Arylalkyl- oder C₇₋₁₉-Alkylarylresten.

In einer *bevorzugten* Ausführungsform der vorliegenden Erfindung haben die Reste in Formel (I) die folgenden Bedeutungen:
- R¹: ist ausgewählt aus Methyl, Ethyl, n-Propyl oder Isopropyl,
- R²: ist ausgewählt aus Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2- Difluorethyl, 2,2,2-Trifluorethyl, 2-Chlor-2-fluorethyl, 2-Chlor,2-difluorethyl, 2,2- Dichlor-2-fluorethyl, 2,2,2-Trichlorethyl, Pentafluorethyl und 1,1,1-Trifluorprop-2-yl; 1,2,2,2-Tetrafluorethyl.
- Y: ist ausgewählt aus der Gruppe bestehend aus (C=O)OR³, CN und (C=O)NR⁴R⁵, wobei R³, R⁴ und R⁵ unabhängig voneinander ausgewählt sind aus Methyl, Ethyl, n-Propyl oder Isopropyl,

In einer besonders bevorzugten Ausführungsform der vorliegenden Erfindung haben die Reste in Formel (I) die folgenden Bedeutungen:
- R¹: ist Methyl,
- R²: ist ausgewählt aus Trifluormethyl oder Difluormethyl
- Y: ist ausgewählt aus der Gruppe bestehend aus (C=O)OR³, wobei R³ Methyl oder Ethyl ist.
Die Durchführung des erfindungsgemäßen Verfahrens erfolgt vorzugsweise innerhalb eines Temperaturbereichs von -20°C bis +150°C, besonders bevorzugt bei Temperaturen von -10°C bis +70 °C.

Das erfindungsgemäße Verfahren wird im Allgemeinen unter Normaldruck durchgeführt. Alternativ ist es jedoch auch möglich, im Vakuum zu arbeiten, um die leicht flüchtigen Dialkylamine zu entfernen.

Die Reaktionszeit ist nicht kritisch und kann, in Abhängigkeit von der Ansatzgröße und Temperatur, in einem Bereich zwischen wenigen Minuten und mehreren Stunden gewählt werden.

Bei der Durchführung des erfindungsgemäßen Verfahrens setzt man 1 Mol des Acrylsäure-Derivats der Formel (II) mit 0.5 Mol bis 3 Mol, vorzugsweise 0.5 Mol bis 1.5 Mol, besonders bevorzugt mit der äquimolaren Menge des Hydrazins der Formel (III) und mit 0.5 Mol bis 50 Mol, vorzugsweise 0.5 Mol bis 20 Mol, besonders bevorzugt mit 1 bis 2 Mol Menge der Carbonylverbindung der Formel (IV) um.

Vorzugsweise wird das im Lösungsmittel gelöste Hydrazin der Formel (III) zusammen mit Carbonylverbindung vorgelegt und das Acrylsäure-Derivat der Formel (II) zugegeben. Die umgekehrte Reihenfolge ist jedoch auch möglich. Idealerweise wird die Reaktion direkt in Überschuss von Carbonylverbindung zum Beispiel in Acetone oder Pinakolon durchgeführt. Vor der Isolierung des Produktes wird zu dem Reaktionsgemisch die Säure zugegeben. Geeignete Säuren sind ausgewählt aus der Gruppe bestehend aus HCl, H₂SO₄, CF₃COOH, CF₃SO₃H, CH₃COOH, besonders bevorzugt sind HCl und H₂SO₄.

Die Acrylsäure-Derivate der Formel (II) sind erhältlich nach den zuvor, im Zusammenhang mit dem Stand der Technik, beschriebenen Verfahren.

Im Zusammenhang mit der vorliegenden Erfindung werden vorzugsweise 2-acylierte Acrylsäure-Derivate der Formel (II) verwendet, die ausgewählt sind aus der Gruppe bestehend aus Ethyl-(2-ethoxymethylen)-4,4-difluormethylacetoacetat, Ethyl-(2-ethoxymethylen)-4,4,4-trifluormethylacetoacetat , Ethyl-(2-ethoxymethylen)-4,4,4-trifluormethylacetonitril.

Die Monoalkyl-Hydrazine der Formel (III) sind im Zusammenhang mit der vorliegenden Erfindung vorzugsweise ausgewählt aus der Gruppe bestehend aus Monomethylhydrazin, Monoethylhydrazin, Monopropylhydrazin, Phenylhydrazin.

Die Carbonylverbindungen der Formel (IV) sind im Zusammenhang mit der vorliegenden Erfindung vorzugsweise ausgewählt aus der Gruppe Acetone, Pinakolone, Benzaldehyd, Benzophenone, Cyclohexanon, Methylethylketone., besonders bevorzug sind Acetone, Pinacolone, Benzaldehyd

Ein weiterer Vorteil des erfindungsgemäßen Verfahrens, ist die Tatsache, dass zur Herstellung von Pyrazolen der Formel 1 wässrige Methylhydrazinlösungen verwendet werden können und nicht zwangsläufig das explosive, auch als Raketentreibstoff verwendete, konzentrierte Methylhydrazin eingesetzt werden muss.

Die Reaktion kann in Substanz oder in einem Lösungsmittel durchgeführt werden. Vorzugsweise wird die Reaktion in einem Lösungsmittel durchgeführt. Geeignete Lösungsmittel sind beispielsweise ausgewählt aus der Gruppe bestehend aus aliphatischen und aromatischen Kohlenwasserstoffen, wie z.B. Wasser, Alkohole (Methanol, Ethanol, Isopropanol), n-Hexan, Benzol oder Toluol, die durch Fluor- und Chloratome substituiert sein können, wie Methylenchlorid, Dichlorethan, Fluorbenzol, Chlorbenzol oder Dichlorbenzol; Ethern, wie z.B. Diethylether, Diphenylether Methyl-tert-butylether, Isopropylethylether, Dioxan, Diglym, Dimethylglycol, Dimethoxyethane (DME) oder THF; Nitrilen wie Methylnitril, Butylnitril oder Phenylnitril; Amide wir Dimethylformamid (DMF) oder N-methlypyrollidon (NMP) oder Mischungen solcher Lösungsmittel geeignet, wobei Acetonitril, Dichlormethan, THF, DME und Ethylacetate, Acetone, Wasser, Ethanol besonders bevorzugt sind.

Nach beendeter Reaktion wird beispielsweise die Lösemitteln entfernt und das Produkt durch Filtration isoliert oder zunächst mit Wasser extrahiert, die organische Phase abgetrennt und das Lösungsmittel durch Destillation entfernt.

Das erfindungsgemäße Verfahren soll in den nachstehenden Beispielen weiter beschrieben werden. Die Beispiele sind jedoch nicht in einschränkender Weise zu interpretieren.

### Herstellungsbeispiele

### Beispiel 1: Ethyl-3-(difluoromethyl)-1-methyl-1H-pyrazol-4-carboxylat

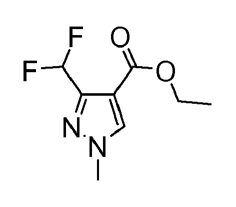

100 ml Aceton und 12 g von Methylhydrazin wurden bei 10°C zusammengemischt und das Gemisch 1 Std bei RT nachgerührt.

58 g (25 mmol) Ethyl 2-(Ethoxymethyliden)-4,4-Difuoro-3-oxobutanoat wurde zugegeben und das Gemisch 3 Std bei RT nachgerührt und dann mit 1 ml 1 10 % HCl versetzt. Das GC zeigte nur ein Isomer. Das Gemisch wurde eingeengt und das Produkt mit kaltem Wasser gewaschen. Ausbeute 48.3 g (94 %).

**¹⁹F-NMR** (CDCl₃): δ =-117,2 (d) ppm.

**¹H-NMR** (CDCl₃): δ= 1,35 (t, 3H); 3,96 (s, 3H); 4,31 (kw, 2H); 7,10 (t, 1H), 8,15 (s, 1H) ppm.

### Beispiel 2: Ethyl 3-(chlorfluormethyl)-1-methyl-1H-pyrazol-4-carboxylat

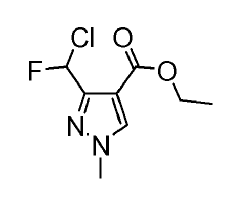

Abweichend von Beispiel 1 wird 2-(Ethoxymethyliden)-4-chlor-4,4-difluor-3-oxobutanoate eingesetzt.

**¹⁹F-NMR** (CDCl₃): δ=-133,8 (d, J=47,5) ppm.

## Patentansprüche

1. Verfahren zum Herstellen von 1-Alkyl-3-haloalkyl-pyrazol-4-carbonsäure-Derivaten der Formel (I) 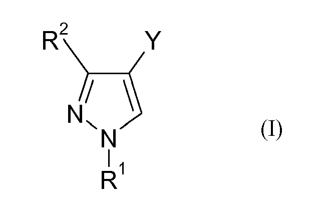 in welcher
R¹ ausgewählt ist aus C₁-C₄-Alkylgruppen,
R² ausgewählt ist aus C₁-C₄-Alkylgruppen, die mit einem, zwei oder drei Halogenatomen, ausgewählt aus F, Cl und Br oder einer CF₃-Gruppe substituiert sein können;
Y ausgewählt ist aus der Gruppe bestehend aus (C=O)OR³, CN und (C=O)NR⁴R⁵, wobei R³, R⁴ und R⁵ unabhängig voneinander ausgewählt sind aus C₁₋₁₂-Alkyl-, C₃₋₈-Cycloalkyl-, C₆₋₁₈-Aryl-, C₇₋₁₉-Arylalkyl- oder C₇₋₁₉-Alkylarylresten oder wobei R⁴ und R⁵ gemeinsam mit dem N-Atom an das sie geknüpft sind einen fünf oder sechsgliedrigen Ring bilden können;
umfassend die Umsetzung eines 2-acylierten oder 2-iminoalkylierten Acrylsäure-Derivats der Formel (II), 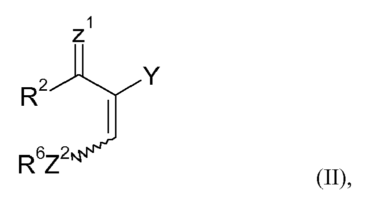 in welcher
Z¹ und Z² unabhängig voneinander ausgewählt sind aus O und S;
R⁶ ausgewählt ist aus C₁₋₁₂-Alkylgruppen;
R² und Y die zuvor beschriebenen Bedeutungen haben;
mit einem N-Alkyl-Hydrazin der Formel (III) 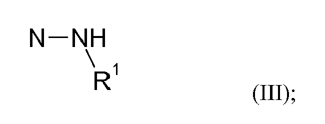 in welcher
R¹ die zuvor beschriebene Bedeutung hat;
in Gegenwart von einer Carbonylverbindung der Formel (IV) 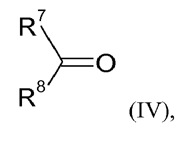 in welcher
R⁷, R⁸ unabhängig voneinander ausgewählt sind aus H, C₁₋₁₂-Alkyl-, C₃₋₈-Cycloalkyl-, C₆₋₁₈-Aryl-, C₇₋₁₉-Arylalkyl- oder C₇₋₁₉-Alkylarylresten

2. Verfahren gemäß Anspruch 1, **dadurch gekennzeichnet, dass** das 2-acylierte Acrylsäure-Derivat der Formel (II) ausgewählt ist aus der Gruppe bestehend aus Ethyl-(2-ethoxymethylen)-4,4-difluormethylacetoacetat, Ethyl-(2-ethoxymethylen)-4,4,4-trifluormethylacetoacetat, Ethyl-(2-ethoxymethylen)-4,4,4-trifluormethylacetonitril.

3. Verfahren gemäß einem der Ansprüche 1 bis 2, **dadurch gekennzeichnet, dass** das N-Alkyl-Hydrazine der Formel (III) ausgewählt ist aus der Gruppe bestehend aus Monomethylhydrazin, Monoethylhydrazin, Phenylhydrazin.

4. Verfahren gemäß einem der Ansprüche 1 bis 3, **dadurch gekennzeichnet, dass** die Carbonylverbindungen der Formel (IV) ausgewählt ist aus der Gruppe bestehend aus Aceton, Pinakolon, Benzaldehyd, Benzophenon.

5. Verfahren gemäß einem der Ansprüche 1 bis 4, **dadurch gekennzeichnet, dass** als Acrylsäure-Derivat der Formel (II) Ethyl-(2-ethoxymethylen)-4,4-difluormethylacetoacetat, als Hydrazin der Formel (III) Methylhydrazin und als Carbonylverbindung der Formel (IV) Aceton verwendet wird.
